# Europäisches Patentamt
## European Patent Office
## Office européen des brevets

(19)

(11) Veröffentlichungsnummer: **0 051 797**
**B1**

(12)

# EUROPÄISCHE PATENTSCHRIFT

(45) Veröffentlichungstag der Patentschrift:
**16.05.84**

(21) Anmeldenummer: **81109074.5**

(22) Anmeldetag: **28.10.81**

(51) Int. Cl.³: **C 09 J 3/14,** C 08 L 33/06,
C 08 K 5/55, C 08 F 4/52,
C 08 F 20/18, A 61 K 6/08,
A 61 B 17/18

(54) Verfahren zum Härten von Reaktionsklebstoffen.

(30) Priorität: **06.11.80 DE 3041843**

(43) Veröffentlichungstag der Anmeldung:
**19.05.82 Patentblatt 82/20**

(45) Bekanntmachung des Hinweises auf die Patenterteilung:
**16.05.84 Patentblatt 84/20**

(84) Benannte Vertragsstaaten:
**AT BE CH DE FR GB IT LI NL**

(56) Entgegenhaltungen:
**AT - B - 221 813
DE - A - 2 321 215
GB - A - 1 129 525
US - A - 4 182 823**

(73) Patentinhaber: **Henkel Kommanditgesellschaft auf Aktien, Postfach 1100 Henkelstrasse 67,
D-4000 Düsseldorf-Holthausen (DE)**

(72) Erfinder: **Ritter, Wolfgang, Dr., Oppelner Weg 3,
D-4000 Düsseldorf 12 (DE)**
Erfinder: **Gruber, Werner, Dr., Franz-Kremer-Strasse 7,
D-4052 Korschenbroich (DE)**

**0 051 797**

## Verfahren zum Härten von Reaktionsklebstoffen

Die Erfindung betrifft ein Verfahren zur Härtung von Reaktionsklebstoffen auf Basis von ethylenische Doppelbindungen enthaltenden Verbindungen mit Boralkyl-Verbindungen, welche an der Luft handhabbar sind als Initiatoren.

Reaktionsklebstoffe, die durch Polymerisation von ethylenischen Gruppen enthaltenden Verbindungen härten, sind seit langem bekannt. Diese lassen sich aus Methacrylsäureestern beziehungsweise Acrylsäureestern der verschiedensten Alkohole herstellen. Zu ihrer Härtung verwendet man meist organische Peroxide beziehungsweise Hydroperoxide zusammen mit weiteren Hilfsmitteln wie Beschleunigern.

Darüber hinaus sind für dentalmedizinische oder chirurgische Anwendungen Binde- und Füllmittel bekannt, die neben Acrylsäure- oder Methacrylsäureestern weitere ethylenisch ungesättigte Verbindungen enthalten und zu deren Härtung Trialkylbor-Verbindungen wie zum Beispiel Triethylbor, Trin-Butylbor und so weiter verwendet werden. Derartige Trialkylbor-Verbindungen weisen aber den Nachteil auf, leicht entzündlich zu sein, so daß ihre Verwendung in einem Verfahren zur Härtung von Klebstoffen erhebliche Schwierigkeiten bereitet. Um diesem Übelstand abzuhelfen, wurde in der deutschen Offenlegungsschrift 23 21 215 vorgeschlagen, die Trialkylbor-Verbindungen mit 0,3 bis 0,9 Mol Sauerstoff umzusetzen, um ihre Selbstentzündlichkeit herabzusetzen.

Weiterhin ist es bekannt, die Selbstentzündlichkeit von Trialkylbor-Verbindungen durch Zugabe von Aminen herabzusetzen.

Durch diese Maßnahmen wird die Zündtemperatur zwar in einen Bereich von 0 bis 70° C verschoben, aber trotzdem bleibt eine erhebliche Unsicherheit bei der Handhabung derartiger Mischungen, wodurch die bisher bekannten Verfahren zur Härtung von Reaktionsklebstoffen mit Bor-Verbindungen aufgrund der erforderlichen Sicherheitsvorkehrungen aufwendig und wenig praxisgerecht insbesondere bei Konstruktivverklebungen sind.

Aufgabe der vorliegenden Erfindung war es daher, ein neues Verfahren zur Härtung von Reaktionsklebstoffen mit Boralkyl-Verbindungen vorzuschlagen, bei dem keine selbstentzündlichen Substanzen verwendet werden und daß trotzdem bei praktikablen Topfzeiten zu guten Verklebungen führt, auch an solchen Materialien, die sich nicht trocknen lassen. Eine weitere Aufgabe der Erfindung war es, dieses Verfahren insbesondere für Klebemittel vorzuschlagen, die im dentalmedizinischen Bereich sowie in der Chirurgie eingesetzt werden, so etwa bei der Verklebung von Knochen beziehungsweise Zähnen oder sonstigen harten lebenden Geweben.

Gegenstand der Erfindung ist somit ein Verfahren zur Härtung von Reaktionsklebstoffen auf Basis von ethylenische Doppelbindungen enthaltenden Verbindungen mit Boralkylen, dadurch gekennzeichnet, daß als Boralkyl 9-Borabicyclo(3,3,1)-nonan oder dessen Additionsprodukte an ethylenisch ungesättigte Verbindungen verwendet werden.

Nach einer ersten Ausführungsform der Erfindung ist 9-Borabicyclo(3,3,1)-nonan ein geeigneter Härter für Reaktionsklebstoffe. Diese Borverbindung besitzt praktisch keine Selbstentzündlichkeit an der Luft. Sie weist eine mit Natriumborhydrit und Litiumaluminiumhydrit vergleichbare Stabilität auf und ist somit ähnlich wie diese Verbindungen zu handhaben. 9-Borabicyclo(3,3,1)-nonan kann aus Cyclooctadien und Natriumborhydrit und Bortrifluorid in an sich bekannter Weise hergestellt werden.

Nach einer weiteren Ausführungsform des erfindungsgemäßen Verfahrens können Additionsprodukte von 9-Borabicyclo(3,3,1)-nonan an ethylenisch ungesättigte Verbindungen eingesetzt werden. Als ethylenisch ungesättigte Verbindungen sind hier denkbar: Buten, Isobuten, Hexen, Cyclohexen, Vinylchlorid, Allylchlorid, Allylamin oder auch Methacrylsäuremethylester, Vinylacetat oder Crotonsäuremethylester.

Von den einzusetzenden Initiatoren verwendet man etwa 0,1 bis 10%, bezogen auf den polymerisierbaren Anteil. Vorzugsweise setzt man etwa 0,5 bis 3%, bezogen auf die eingesetzten Monomeren, ein. Es kann vorteilhaft sein, aus dem Initiator zunächst ein Voraddukt mit einem ethylenisch ungesättigten Monomeren herzustellen.

Als polymerisierbare Bestandteile des erfindungsgemäßen Reaktionsklebstoffes kommen zahlreiche Verbindungen in Frage, die ethylenische Doppelbindungen enthalten, zum Beispiel die Methacrylsäureester von monovalenten Alkoholen, also etwa (Meth)acrylsäuremethylester, (Meth)acrylsäureethylester, (Meth)acrylsäurebutylester und (Meth)acrylsäureethylhexylester, die (Meth)arcylsäureester von polyvalenten Alkoholen, wie zum Beispiel Ethylenglycol, Diethylenglycol, Polyethylenglycol und Trimethylolpropan, die Di- und Mono(meth)acrylsäureester von Glycerin, die Dimethacrylsäureester von Tri- und Tetraethylenglykol von Di-, Tri-, Tetra- und Pentapropylenglycol, die Di(meth)acrylsäureester von ethoxyliertem oder propoxyliertem Diphenylolpropan. Auch kommen (Meth)acrylsäureester von Alkoholen in Frage, die sich vom Dihydroxymethyltricyclodecan ableiten oder auch solche, die auf Basis von Tricyclodecan hergestellt worden sind, wobei 2 alkoholische Funktionen im Ringsystem durch Umsetzung mit Dicarbonsäuren wie Maleinsäure oder auch Cyclohexandicarbonsäure oder Terephthalsäure verlängert sind. Des weiteren sind einsetzbar Umsetzungsprodukte von Epoxiden (Propylenoxid, Ethylenoxid) mit zum Beispiel Diphenylolpropan. Ferner sind geeignet die (Meth)acrylsäureester, die durch Umsetzung von Diisocyanaten mit Hydroxyalkyl(meth)acrylsäureester erhältlich sind.

Schließlich können den neuen Systemen auch weitere polymerisierbare Verbindungen vorzugsweise in untergeordneter Menge zugefügt werden, wie zum Beispiel Vinylacetat, Crotonsäure- und Maleinsäureester, Styrol, Divinylbenzol und dergleichen mehr.

Außerdem kommen in Frage 2-Acryloyloxyethylphosphat, 2-Methacryloyloxyethylphosphat, Bis-2-acryloyloxyethylphosphat, Bis-2-methacryloyloxyethylphosphat. Tris-2-acryloyloxyethylphosphat, Tris-2-methacryloyloxyethylphosphat und Säureamide wie etwa Dimethylenbis(meth)-acrylamid, Tetramethylenbis(meth)acrylamid, Tri(meth)acryloyldiethylentriamin und dergleichen mehr.

Weiterhin ist es häufig zweckmäßig, Polymerisate wie Polymethylmethacrylat, Polyvinylacetat, chlorsulfoniertes Polypropylen oder dergleichen zur Verstärkung und gleichzeitig als Verdickungsmittel zuzusetzen, um die Verarbeitung der Klebstoffe zu erleichtern.

In vielen Fällen ist es zweckmäßig, noch weitere Hilfsstoffe wie Füllstoffe, zum Beispiel Quarzmehl oder dergleichen zuzugeben. Auch kann es in manchen Fällen zweckmäßig sein, mit geeigneten Farbstoffen einzufärben.

Das erfindungsgemäße Verfahren zeichnet sich durch eine hohe Härtungsgeschwindigkeit der Reaktionsklebstoffe bei Raumtemperatur aus und liefert nach kurzer Zeit gute Festigkeiten auf einer Vielzahl von unterschiedlichen Oberflächen. Hervorzuheben ist insbesondere, daß auch auf feuchterer Oberfläche eine schnelle und gute Haftung erzielt wird. Das erfindungsgemäße Verfahren kann demzufolge bei der Härtung von Konstruktionsklebstoffen zur Verklebung von Metallen, Holz, Glas, Keramik und Kunststoff eingesetzt werden.

Erfindungsgemäß lassen sich weiterhin Verklebungen von hartem Gewebe insbesondere Knochen oder auch Zähnen mit sich selbst oder mit metallischen Oberflächen durchführen.

Aus AT-B-221 813 sind Massen auf Basis ethylenische Doppelbindungen enthaltender Systeme sowie Borverbindungen als Polymerisationsinitiatoren bekannt, bei denen man Borverbindungen aus der Gruppe der Monoalkylborsäuren und deren Anhydriden verwendet, die praktisch keine Selbstentzündlichkeit an der Luft mehr besitzen. Diese Borverbindungen unterscheiden sich erheblich von den erfindungsgemäß eingesetzten Borverbindungen, und es werden auch keine Reaktionsklebstoffe beschrieben.

## Beispiel 1

In einem Becherglas wurden 4 g Polymethacrylsäuremethylester (handelsübliches Pulver, Glastemperatur 180°C) in 4,5 g Methacrylsäuremethylester und 0,5 g Methacrylsäure unter Rühren gelöst. Unter weiterem intensiverem Rühren wurden 0,3 g 9-Borabicyclo(3,3,1)-nonan zugegeben.

Die Mischung hat eine Topfzeit von etwa 5 min. An Prüfkörpern aus Buchenholz wurde die durchschnittliche Zugscherfestigkeit nach 24 Stunden bestimmt: 15 N/mm² (DIN 68 602). Weiter wurde an sandgestrahlten und entfetteten Eisenblechen ebenfalls nach 24 Stunden die Zugscherfestigkeit gemessen: 24 N/mm² (DIN 53 281/3).

## Beispiele 2 bis 5

In diesen Mischungen wurden als Initiator jeweils 0,3 g 9-Borabicyclo(3,3,1)-nonan und als Verdicker 4 g Polymethylmethacrylsäureester verwendet. Dazu wurden verschiedene Monomere gegeben und Verklebungen an Buchenholz- und Eisenprüfkörpern durchgeführt. In der nachfolgenden Tabelle sind in Abhängigkeit von der laufenden Nummer des Beispiels die eingesetzten Monomeren und die Topfzeit aufgeführt. Es folgen die gefundenen durchschnittlichen Werte für die Zugscherfestigkeit nach 24 Stunden an Eisen und Buchenholz.

Tabelle

| Bei-spiel | Monomere | Topfzeit min | Zugscherfestigkeit/ N/mm² | |
|---|---|---|---|---|
| | | | Fe | Holz |
| 2 | 4,5 g Methacrylsäuremethylester | 15 | 15 | 8 |
| 3 | 4,5 g Methacrylsäuremethylester 0,5 g Ethylenglycoldimethacrylsäureester | 25 | 15 | 8 |
| 4 | 3,0 g Methacrylsäuremethylester 0,5 g Ethylenglycoldimethacrylsäureester 1,5 g Acrylsäurebutylester | 20 | 15 | 9 |
| 5 | 4,5 g Methacrylsäuremethylester 0,5 g Ethylenglycolmonomethacrylsäureester | 20 | 18 | 11 |

**0 051 797**

### Herstellung eines Initiators

Unter Ausschluß von Sauerstoff wurden 12 g 9-Borabicyclo(3,3,1)-nonan in 100 ml wasserfreiem und entgastem Tetrahydrofuran gelöst. Diese Lösung wurde unter weiterem Ausschluß von Sauerstoff mit 8,6 g entgastem Methacrylsäuremethylester versetzt. Während des Zutropfens wurde eine exotherme Reaktion beobachtet. Nach Abklingen der Wärmeentwicklung wurde das überschüssige Tetrahydrofuran im Vakuum abgezogen. Der so erhaltene Initiator wird in den folgenden Beispielen verwendet.

### Beispiel 6

Zu einer Lösung von 4 g Polymethacrylsäuremethylester in 4,5 g Methacrylsäuremethylester und 0,5 g Methacrylsäure wurden 0,5 g des wie vorstehend hergestellten Initiators gegeben und nach Zusatz von 0,35 g Benzoesäuremethylester gut gemischt.

Mit diesem Klebstoff wurden Prüfkörper aus Buchenholz miteinander verklebt und nach 24 Stunden die Zugscherfestigkeit bestimmt. Sie betrug durchschnittlich 13 N/mm² (DIN 68 602).

Weiterhin wurden sandgestrahlte und entfettete Prüfkörper aus Eisen miteinander verklebt und nach 24stündiger Lagerung die Zugscherfestigkeit bestimmt. Sie betrug durchschnittlich 25 N/mm² (DIN 53 281/3).

### Beispiel 7

Es wurden intensiv und schnell miteinander gemischt 3 g chlorsulfoniertes Polypropylen (1,7% S; 1,85% Cl), 6,2 g Methylmethacrylsäureester und 0,8 g Methacrylsäure sowie 0,5 g des wie vorstehend beschriebenen Starters. Dazu wurden noch 0,35 g Benzoesäuremethylester hinzugefügt. In der nach DIN 68 602 sowie DIN 53 281/3 vorgeschriebenen Weise wurden entsprechend behandelte Prüfkörper aus Buchenholz und Eisen miteinander verklebt.

Es wurden die durchschnittlichen Zugscherfestigkeiten bestimmt: Buchenholz 10 N/mm² und Eisen 27 N/mm².

### Beispiel 8

Frischextrahierte Humanbackenzähne wurden zur Entfernung von Geweberesten 4 Minuten in 3gewichtsprozentigem $H_2O_2$ gekocht. Zur Fixierung wurden die Zähne mit den Wurzeln mittels eines handelsüblichen Zweikomponenten-Klebstoffs auf Basis von anpolymerisiertem Methacrylsäuremethylester in Buchenholzklötze eingeklebt. Die Kronen der Zähne wurden plangeschliffen. Die Schnittfläche bestand aus Zahnschmelz und zum geringeren Anteil aus Dentin. Sie wurde zunächst mit Trichlorethylen entfettet, dann mit einer wäßrigen Lösung von 50prozentiger Phosphorsäure und anschließend mit Hydroxyethylmethacrylsäureester bepinselt und nach 5 Minuten mit Zellstoff abgewischt. Jeweils 2 Prüfkörper wurden mit der im Beispiel 1 beschriebenen Klebstoffmischung unter leichtem Druck fixiert und nach 24 Stunden unter Zugbedingungen zerrissen. Das Aushärten erfolgte

a)  an der Luft,
b)  in Wasser bei 37° C.

Zerrissen wurde nach 24 Stunden. Die angegebenen Zugfestigkeiten sind die Mittelwerte von jeweils 6 Messungen.

a)  6,4 N/mm²,
b)  6,8 N/mm².

### Beispiel 9

In der gleichen Weise, wie im Beispiel 8 beschrieben, wurden Menschenzähne mit einem Klebstoff aus 4, 5 g Methacrylsäuremethylester, 0,5 g Hydroxyethylmethacrylsäure, 4 g Polymethacrylsäuremethylester und 0,3 g 9-Borabicyclo(3,3,1)-nonan verklebt und folgende Zugfestigkeiten ermittelt:

a)  8,3 N/mm²,
b)  5,1 N/mm².

### Beispiel 10

Mit der Klebstoffmischung aus Beispiel 7 wurden, wie im Beispiel 8 beschrieben, Humanzähne verklebt und die folgenden Zugfestigkeiten ermittelt:

a)  5,1 N/mm²,
b)  5,1 N/mm².

4

**0 051 797**

Beispiel 11

Aus 40 g Methacrylsäuremethylester, 10 g Ethylenglycoldimethacrylsäureester, 10 g Bis-2-methacryloxyethylphosphorsäureester, 50 g Polymethylmethacrylat und 1,8 g 9-Borabicyclo(3,3,1)-nonan wurde — wie im Beispiel 1 beschrieben — eine Klebstoffmischung hergestellt und Menschenzähne in Analogie zum Beispiel 8 verklebt. Folgende Zugfestigkeiten wurden bestimmt:

a)  10,3 N/mm²,
b)  11,3 N/mm².

Beispiel 12

Entfettete, gewebefreie Kompaktastücke (aus der Hülle eines Röhrenknochens vom Kalb) mit den Maßen 6 · 0,8 · 0,6 cm (Kieler Knochenspan) wurden ohne weitere Vorbehandlung jeweils mit den Flächen 0,6 · 0,8 cm mit dem Klebstoff aus Beispiel 11 unter leichtem Druck verklebt. Das Aushärten erfolgte

a)  an der Luft,
b)  in Wasser bei 37° C.
Zerrissen wurde nach 24 Stunden. Die angegebenen Zugfestigkeiten sind Mittelwerte von jeweils 6 Messungen.

a)  11 N/mm²,
b)  10,5 N/mm².

**Patentansprüche**

1. Verfahren zur Härtung von Reaktionsklebstoffen auf Basis von ethylenische Doppelbindungen enthaltenden Verbindungen mit Boralkylen, dadurch gekennzeichnet, daß als Boralkyl 9-Borabicyclo-(3,3,1)-nonan oder dessen Additionsprodukte an ethylenisch ungesättigte Verbindungen verwendet werden.

2. Verfahren nach Anspruch 1, dadurch gekennzeichnet, daß die Boralkyle in einer Menge von 0,1 bis 10 Gew.-%, bezogen auf die Gesamtmenge der polymerisierbaren Anteile eingesetzt werden.

**Claims**

1. A process for curing reactive adhesives based on compounds containing ethylenic double bonds using boron alkyls, characterized in that 9-borabicyclo(3,3,1)-nonane or adducts thereof with ethylenically unsaturated compounds are used as the boron alkyl.

2. A process as claimed in claim 1, characterized in that the boron alkyls are used in a quantity of from 0,1 to 10% by weight, based on the total quantity of polymerizable fractions.

**Revendications**

1. Procédé de durcissement d'adhésifs réactifs à base de composés contenant des doubles liaisons éthyléniques, avec des bores alcoylés, caractérisé en ce qu'on utilise comme bore alcoylé du 9-borabicyclo(3,3,1)-nonane ou ses produits d'addition sur des composés éthyléniquement insaturés.

2. Procédé selon la revendication 1, caractérisé en ce qu'on utilise les bores alcoylés en une quantité de 0,1 à 10% en poids par rapport à la quantité totale des fractions polymérisables.